# EUROPEAN PATENT SPECIFICATION

(11) **EP 2 131 824 B1**
(45) Date of publication and mention of the grant of the patent: **24.09.2014**
(21) Application number: 08726047.7
(22) Date of filing: 26.02.2008
(51) Int. Cl.: A61K 9/70, A61K 31/167

(54) **FAST-DISSOLVING/DISINTEGRATING FILM PREPARATION HAVING HIGH PROPORTION OF ACTIVE AGENT**
SCHNELL AUFLÖSENDE/ZERFALLENDE FILMZUBEREITUNG MIT HOHEM WIRKSTOFFANTEIL
PRÉPARATION DE FILM SE DISSOLVANT/DÉSINTÉGRANT RAPIDEMENT, QUI PRÉSENTE UNE PROPORTION ÉLEVÉE D'AGENT ACTIF

(30) Priority: 05.03.2007 US 714007
(43) Date of publication of application: 16.12.2009
(60) Divisional application: 11195471.5
(73) Proprietor: McNeil-PPC, Inc., Skillman, NJ 08858 (US)
(72) Inventor: NG, Charlene, Scotch Plains, New Jersey 07076 (US); MODY, Seema, Fort Washington PA 19034 (US); GHAIM, Josh, Princeton, New Jersey 08540 (US)
(74) Representative: Kirsch, Susan Edith
(86) International application number: PCT/US2008/002462
(87) International publication number: WO 2008/108940

(56) References cited:
- WO-A-00/18365
- WO-A-03/030881
- WO-A-2004/019885
- WO-A-2004/043440
- WO-A-2004/096174
- WO-A-2005/105047

## Description

### TECHNICAL FIELD

The invention relates generally to film preparations, and more particularly, to a fast-dissolving or fast-disintegrating film preparation having a high proportion (i.e., at least about 40%) of active ingredient and related methods for its preparation.

### BACKGROUND OF THE INVENTION

Various dissolvable films are available for the delivery of active agents such as pharmaceuticals, nutritional supplements (e.g., vitamins and minerals), and cosmetic agents (e.g., tooth-whiteners, breath fresheners, etc.). Often, such films are adapted for oral administration and intended to quickly dissolve or disintegrate within the oral cavity. Other films are adapted for topical administration.

A shortcoming of known film preparations is the relatively low dosage of active ingredients that can be included in the film. In order to achieve the desired flexibility, strength, integrity, and ease of handling, film-forming polymers typically comprise the majority of the weight of a dried film, with other ingredients (e.g., plasticizers, flavorings, colorants, etc.) also comprising a substantial proportion of the dried film. This leaves a relatively small proportion of the dried film available for the active agent(s). Thus, the administration of a high dosage of an active agent may require the use of an unacceptably large film or the administration of multiple smaller dosages of the active agent in a standard-sized film.

An additional shortcoming of known film preparations is the incompatibility of high dosages of many active agents and common film-forming polymers. In some cases, this is due, in part, to the shortcoming above, i.e., a relatively high proportion of film-forming polymer is needed to produce a film preparation having the desired integrity, strength, and flexibility. In other cases, the incompatibility of high dosages of active agents and film-forming polymers may be due to an actual chemical incompatibility. As a result of these shortcomings, film preparations have not been a viable form of administration of some active agents, particularly where a relatively high dosage of the active agent is required.

For example, U.S. Patent No. 6,709,671 to Zerbe et al. describes a water-soluble film for oral administration comprising a water-soluble polymer, a surfactant, and an active agent, wherein the polymer comprises between 20% and 75% and the active agent comprises 0.01 % to 20% of the weight of the dried film. Such a film is not suitable for the administration of a high dosage (e.g., 50 mg) of an active ingredient, since the film would need to weigh 250 mg in order to contain 50 mg of the active ingredient, even if the active comprised 20% of the dried weight of the film. Such a film would be quite large and unsuitable for oral administration or routine or discrete topical administration.

U.S. Patent No. 6,906,043 to Awamura et al. claims to describe a rapidly-soluble film preparation comprising a drug, a polymer, and a saccharide, wherein the drug comprises 0.01% to 50% and the polymer comprises between 20% and 90% of the preparation, by weight. However, in all of the examples of film preparations provided, the drug (either nilvadipine, indomethacin, or fenoterol hydrobromide) never comprises more than 20% of the weight of the dried film and the polymers used always comprise at least 58% of the weight of the dried film, and usually comprise more than 75% of its weight.

U.S. Patent Application Publication No. 20050186257 to Manegold et al. describes a method for manufacturing a dissolvable film comprising at least about 18% of an active ingredient having a water solubility of less than about 1 g / 4 mL (e.g., caffeine). However, in none of the examples provided does the active ingredient comprise more than about 20% of the dry weight of the film.

To this extent, a need exists for a fast-dissolving or fast-disintegrating film preparation suitable of oral or topical administration comprising a high proportion of active agent, such that the film preparation provides a relatively high dosage of active agent.

### SUMMARY OF THE INVENTION

The invention provides a fast-dissolving or fast-disintegrating film preparation having a high proportion (i.e., at least about 40%) of active agent(s) and related methods for its preparation. In one embodiment, the active agent is a pharmaceutical and the film comprises between about 10% and about 40% pullulan as a primary or sole water-soluble polymer.

A first aspect of the invention provides a fast-dissolving or fast-disintegrating film comprising: an active agent comprising at least about 40% of the film, by weight; at least one water-soluble polymer comprising between about 10% and about 40% of the film, by weight, wherein the active agent is a poorly-water-soluble solid at room temperature.

A second aspect of the invention provides a fast-dissolving or fast-disintegrating film comprising: an active agent comprising at least about 40% of the film, by weight; and pullulan as a sole or primary water-soluble polymer comprising between about 10% and about 40% of the film, by weight, wherein the active agent is a poorly-water-soluble solid at room temperature.

A third aspect of the invention provides a method for preparing a fast-dissolving or fast-disintegrating film comprising a high dose of active agent, the method comprising: dissolving in a quantity of water a quantity of at least one water-soluble polymer comprising between about 10% and about 40% of the dry film, by weight; adding to the resulting solution a quantity of at least one active agent comprising at least about 40%, optionally about 50%, or optionally about 60% of the dry film, by weight; casting the resulting suspension on a supporting substrate; and drying the cast suspension to form a film.

The illustrative aspects of the present invention are designed to solve the problems herein described and other problems not discussed, which are discoverable by a skilled artisan.

### DETAILED DESCRIPTION OF THE INVENTION

The compositions of the present invention can comprise, consist of, or consist essentially of the essential elements and limitations of the invention described herein, as well any of the additional or optional ingredients, components, or limitations described herein.

All percentages, parts and ratios are based upon the total weight of the dry film composition of the present invention, unless otherwise specified. All such weights as they pertain to the listed ingredients are based on the dry weight of the composition, unless otherwise specified.

The term "safe and effective" as used herein means an amount of a compound or composition such as a topical or system active sufficient to significantly induce a positive benefit, for example, a teeth whitening, antimicrobial and/or analgesic benefit, including independently the benefits disclosed herein, but low enough to avoid serious side effects, i.e., to provide a reasonable benefit to risk ratio, within the scope of sound judgment of the skilled artisan.

As indicated above, the invention provides a fast-dissolving or fast-disintegrating film preparation having a high proportion (i.e., at least about 40%, or optionally at least about 50%, or optionally at least 60% or optionally at least 70% of the dry film, by weight) of the active ingredient such that the active ingredient remains safe and effective.

Optionally, the films of the present invention are thin films. As used herein, the term "thin" means film thicknesses of from about 25µm to about 250µm, optionally from about 40µm to about 200µm or optionally from about 80µm to about 180µm.

As used herein, the terms "active," "active agent," and "active ingredient" are used interchangeably and are meant to refer to a substance intended to be delivered, the substance being capable of imparting a desired action or effect. Such substances include, but are not limited to, pharmaceuticals, medicaments, drugs, therapeutic agents, diagnostic agents, cosmetic agents, nutritional supplements and mixtures thereof. More specifically, without limiting the scope of the invention, such substances include pain relievers, cough and cold ingredients, antiinflammatories, antibiotics, sedatives, stimulants, antihistamines, antiallergenics, diuretics, expectorants, hormones, antipsychotics, narcotics and mixtures thereof.

The terms "active," "active agent," and "active ingredient" include unmodified forms or separately processed forms of the active (such as encapsulated or granulated forms). The encapsulated and/or granulated forms are generally used to provide special delivery characteristics prior to film preparation. The special delivery characteristics may include, but are not limited to taste masking, sustained release, or targeted release (for example targeted release in the latter part of the intestinal tract) properties or a combination of these properties.

As used herein, the term "taste masking", means a perceived reduction of an undesirable taste that would otherwise be present.

Suitable taste masking agents include, but are not limited to, sodium bicarbonate; ion exchange resins such as those described in US 7,067,116 to Bess et al.; cyclodextrins capable of forming inclusion compounds such as those described in US 6,942,848 to Nelson et al.; adsorbent substances such as the silicates described in US 4,753,800 to Mozda and US 5,622,980 to Caldwell et al.; and mixtures thereof. Useful taste masking agents are also described in US 4,851,226 to Julian et al.; US 5,405,617 to Gowan et al.; and US 4,7864,375 to Paradissis (together with US 3,037,911 to Stoyle et al.; and US 3,080,292, 3,080,293 and 3,279,994 to Koff).

As used herein, the terms "sustained release", "targeted release", and "controlled release" refer to delivery systems or formulations for delivering oral actives, characterized, for example, by the complete or partial covering or encapsulation of the active ingredient with a coating used to control the rate and/or location of the release of the active ingredients of the oral formulation.

The targeted release of the active into the small intestine is achieved by coating the active with a material impervious or substantially impervious to the acidic environment of the stomach, which has a pH of about 2 to 3, yet soluble in the more neutral environment (i.e., pH greater than about 5) of the duodenum and small intestine. This controlled release prevents the active ingredient from being degraded due to exposure to the highly acidic environment of the stomach and allows the maintenance of therapeutic blood levels of the active ingredients sustained or steady release of the active over time - eliminating the spike and collapse pattern associated with less sophisticated medication systems that release their active ingredients more rapidly into the digestive system.

The sustained or controlled release of the active over the course of its travel through the duodenum and small intestine can be achieved by any of a number of slow dissolution technologies. These include, but are not limited to: encapsulation or microencapsulation of active in coatings of variable thickness, each with a different dissolution pattern; encapsulation of the active within a material matrix that dissolves slowly in the neutral environment of the duodenum and small intestine; and binding of the active with bioadhesives which adhere to the wall of the small intestine, for the purpose of providing sustained or targeted release. Such controlled release systems are described, for example, in US 6,124,477 to Harris; US 5,783,212 to Fassihi et al.; US 5,415,878 to Newton et al.; US 5,225,212 to Martin et al.; US 5,133,974 to Paradissis et al; US 4,695,467 to Uemura et al.; US 4,610,870 to Jain et al.; US 4,259,314 to Lowey; US 4,309,404 to DeNeale et al.; US 4,248,857 to DeNeale et al.; and US 4,140,255 to Weiler.

Detailed descriptions of various coating or encapsulation techniques such as, for example, fluidized bed granulation, hot-melt extrusion, dipping, spinning disk, and emulsion gelation can be found in US 7,048,948 to Carlo et al.; US 2006094097 to Becker et al.; US 4,675,140 to Sparks et al.; US 4,123,206 to Dannelly; US 3,423,489 to Arens et al. and US 20050089548 to Virgalitto et al..

Examples of coating materials useful herein include, but are not limited to, polymethacrylates, ethylcellulose, hydroxypropyl methylcellulose ,cellulose acetate phthalate, hydroxypropyl methylcellulose phthalate, polyvinyl acetate phthalate, carboxymethylethylcellulose, copolymers of methacrylic acid and methacrylic acid methyl esters, such as Eudragit®L 12,5 or Eudragit®L 100 (Rohm Pharma), water based dispersions such as Aquateric® (FMC Corporation), Eudragit®L 100-55 (Rohm Pharma) and Coating CE 5142 (BASF), and those containing water soluble plasticizers such as Citroflex® (Pfizer). A more detailed discussion of useful Eudrgit compounds can be found in US 2005196358 to Georgiades et al.

More detailed information concerning the various materials, equipment and processes useful in preparing coated actives may be found in: Pharmaceutical Dosage Forms: Tablets, eds. Lieberman et al. (New York: Marcel Dekker, Inc., 1989), Ansel et al., Pharmaceutical Dosage Forms and Drug Delivery Systems, 6.sup.th Ed. (Media, Pa.: Williams & Wilkins, 1995) and in US Patent 7,063,748 to Talton.

As used herein, the terms "fast-dissolving" and "fast-disintegrating" mean capable of dissolving or disintegrating, respectively, within about one minute, optionally within about 30 seconds or optionally within about 20 seconds, within the oral cavity of an individual, on a moist surface of an individual's skin, or in an aqueous liquid, but be substantially non-dissolving and non-disintegrating in nonaqueous environments. As used herein, "individual" shall include any mammal, including a human.

As used herein, "poorly-water-soluble" shall mean a solubility of less than about 0.5 g/mL in water at room temperature.

As used herein, "room temperature" shall mean an ambient temperature of from about 20ºC to about 25ºC.

As used herein, "dry" shall mean a moisture content of less than about 15% by weight, more preferably between about 3% and about 12% by weight, and even more preferably between about 5% and about 10% by weight.

Surprisingly, it has been discovered that a thin, fast-dissolving or fast-disintegrating film having a high proportion (i.e., at least about 40%, optionally at least about 45% or optionally at least about 50% or optionally at least about 60% of the dry film, by weight) of active agent may be prepared. In certain embodiments, the active agent is a poorly-water-soluble, solid at room temperature. Optionally, the concentration of the active agent can be from about 40 % to about 90%, optionally about 45% to about 85%, optionally from about 50% to about 80% by weight.

Examples of suitable poorly-water soluble solid actives can be found in US patent 5,484,606 to Dhabhar; US patent 6,638,537 to Dennis et al. and US Patent. 4,522,828, to Sunshine et al.

Films according to the invention are particularly useful in administering active agents that require a high dose to provide their desired effects. For example, ibuprofen, which is practically water-insoluble, requires a dose of at least about 50 mg to be effective. Thus, to be administered in a typical orally-consumable film weighing about 100 mg, the film must comprise about 50% ibuprofen.

Other active agents may require even higher dosages to be effective. For example, acetaminophen, which is only very slightly soluble in water, requires a dose of about 80 mg to be effective. Thus, a 100 mg film must comprise about 80% acetaminophen to be effective in a single dosage.

Without being limited by theory, the water soluble polymer provides sufficient flexibility, strength, and integrity to the film so it can be administered orally or topically. The water soluble polymer comprises between about 10% and about 40%, optionally from about 10% and about 30%, optionally from about 12% and about 18% or optionally 15% of the dry the film, by weight, are also included. A particularly preferred water-soluble polymer is pullulan. Other suitable water-soluble polymers useful in practicing the invention include hydroxypropylmethyl cellulose, hydroxyethyl cellulose, hydroxypropyl cellulose, methyl cellulose, polyvinylpyrrolidone, carboxymethyl cellulose, polyvinyl alcohol, sodium alginate, polyethylene glycol, polyethylene oxide, tragacanth gum, guar gum, acacia gum, arabic gum, polyacrylic acid, methylmethacrylate copolymers, carboxyvinyl polymer, amylose, high amylose starch, hydroxypropylated high amylose starch, dextrin, pectin, chitin, chitosan, levan, elsinan, collagen, gelatin, zein, gluten, soy protein isolate, whey protein isolate, casein, and mixtures thereof.

Films according to the invention may include additional ingredients, such as coloring agents and flavoring agents, as will be recognized by one skilled in the art. It may also contain fats and surfactants for the in-situ emulsification of the active ingredients. However, films according to the invention are preferably free of or substantially free of modified starches. As used herein, the phrase "substantially free of modified starches" means modified starches are present in the films of the present invention at concentrations of less than about 1%, optionally less than about 0.5%, or optionally less than about 0.01%, or optionally at about 0% of the dry film, by weight. Although commonly used as film-formers, modified starches typically require a high amount of plasticizer in order to provide the film with sufficient flexibility. Often, films containing modified starches include as much as 50% plasticizer to provide such flexibility. Given the high proportion of active agent in films according to the invention, such quantities of plasticizer are unacceptably high. Modified starches include, for example, corn starches, modified tapioca starches, acid hydrolyzed corn starches, acid hydrolyzed potato starches, enzyme hydrolyzed corn starches, enzyme hydrolyzed potato starches, hypochlorite-oxidized starches, acid-thinned starches, ethylated starches, cross-bonded starches, hydroxypropylated tapioca starches, hydroxypropylated corn starches, pregelatinized modified starches, and combinations thereof.

Plasticizers or plasticizing agents generally constitute between about 0% to about 20% by dry weight of the film, preferably about 2% to about 10% by dry weight. The softeners can include plasticizers containing, for example, sorbitol and other polyols, glycerin, polyethylene glycol, propylene glycol, hydrogenated starch hydrolysates, corn syrups, other like material or combinations thereof.

Such additional ingredients as discussed above as well as others useful herein are disclosed in further detail in US 5,948,430 to Zerby et al.; US 6,596,298 to Leung et al.; US 6,656,493 to Dzija et al.; and US 20060024425 to Barkalow et al.

Films according to the invention may be prepared by any number of methods, although casting is a preferred method. For example, once a suspension containing the active agent(s) and water-soluble polymer(s) is prepared, as will be described in greater detail below, the suspension may be cast onto a substrate, such as polyester, using knife, bar, or extrusion die coating methods, and dried to form a film. Preferably, films according to the invention are dried to a moisture content of less than about 15%, by weight, more preferably to between about 3% and about 12%, and even more preferably to between about 5% and about 10%.

Once cast and dried, films according to the invention preferably have a thickness between about 25 microns and about 250 microns, although the thickness will depend, in part, on the desired administration. In addition, films according to the invention may be composed of a single layer or multiple layers. In a multi-layer film according to the invention, the total thickness of all layers is preferably between about 25 microns and about 250 microns. Such methods of preparation are described in further detail in previously incorporated by reference patent US 6,596,298.

The invention will now be described more fully with reference to the following examples, which are provided for purpose of illustration and explanation only and are not intended to limit the invention.

### Example 1 - 50% active, 35% polymer

| | **Component** | **mg/film** | **% w/w dry** | **% w/w wet** |
|---|---|---|---|---|
| | purified water | 0 | 0 | 68.1896 |
| | acesulfame K | 0.3169 | 0.3169 | 0.1008 |
| | sucralose | 5.8095 | 5.8092 | 1.8479 |
| | ibuprofen | 50.0000 | 49.9975 | 15.9044 |
| polymer mix | xanthan gum | 0.1585 | 0.1584 | 0.0504 |
| | locust bean gum | 0.1585 | 0.1584 | 0.0504 |
| | carrageenan | 0.8099 | 0.8098 | 0.2576 |
| | pullulan | 34.3363 | 34.3346 | 10.9220 |
| flavor / color mix | glycerin | 0.8803 | 0.8802 | 0.2800 |
| | polysorbate 80 | 0.6725 | 0.6725 | 0.2139 |
| | atmos 300 | 0.6725 | 0.6725 | 0.2139 |
| | neobee 1053 | 1.3486 | 1.3485 | 0.4290 |
| | cherry flavor | 4.7535 | 4.7533 | 1.5120 |
| | FD&C red #40 | 0.0880 | 0.0880 | 0.0280 |
| Total | | 100.00493 | 100 | 100 |

The film of Example 1, is prepared as follows. Acesulfame K and sucralose are added to the water and dissolved. Optionally, the purified water may be preheated to between 40°C and 50°C. In addition, a solvent comprising water and an alcohol may optionally be used. A preferred alcohol for use in orally-consumable films is ethanol. The polymer mix ingredients are premixed, added, and mixed. The flavor / color mix ingredients are then added, followed by the ibuprofen. The resulting suspension is then cast on an appropriate substrate and dried to produce a thin, stand alone film. The film is then cut to yield individual film (or strip) dosages containing 50 mg of ibuprofen.

### Example 2 - 77% active, 16% polymer

| | **Component** | **mg/film** | **% w/w dry** | **% w/w wet** |
|---|---|---|---|---|
| | purified water | 0 | 0 | 49.7678 |
| | acesulfame K | 0.1516 | 0.1465 | 0.0736 |
| | sucralose | 2.7788 | 2.6850 | 1.3487 |
| | acetaminophen | 80.0000 | 77.2987 | 38.8288 |
| polymer mix | xanthan gum | 0.0758 | 0.0732 | 0.0368 |
| | locust bean gum | 0.0758 | 0.0732 | 0.0368 |
| | carrageenan | 0.3874 | 0.3743 | 0.1880 |
| | pullulan | 16.0000 | 15.4597 | 7.7658 |
| flavor / color mix | glycerin | 0.4211 | 0.4068 | 0.2044 |
| | polysorbate 80 | 0.3217 | 0.3108 | 0.1561 |
| | atmos 300 | 0.3217 | 0.3108 | 0.1561 |
| | neobee 1053 | 0.6451 | 0.6233 | 0.3131 |
| | mint flavor | 2.2737 | 2.1969 | 1.1036 |
| | green #3 | 0.0421 | 0.0407 | 0.0204 |
| Total | | 103.49457 | 100 | 100 |

A fast-dissolving, thin film containing 80 mg of acetaminophen is prepared using a method similar to that described above with respect to Example 1. In preparing formulation of this example the acetaminophen can take the form of non-micronized acetaminophen, micronized acetaminophen or preformed taste-masked acetaminophen particles (e.g., acetaminophen coated with ethylcellulose, methylcellulose, polymethyacrylates, polyvinyl alchols, pectin, polyvinylpyrrolidone, various fats and waxes, long chain fatty acids such as stearic acid, or a mixtures thereof).

### Example 3 - 81 % active, 16% polymer

| | **Component** | **mg/film** | **% w/w dry** | **% w/w wet** |
|---|---|---|---|---|
| | purified water | 0 | 0 | 51.0190 |
| | acesulfame K | 0.1516 | 0.1540 | 0.0754 |
| | sucralose | 2.7788 | 2.8228 | 1.3826 |
| | acetaminophen | 80.0000 | 81.2662 | 39.8050 |
| polymer mix | xanthan gum | 0.0758 | 0.0770 | 0.0377 |
| | locust bean gum | 0.0758 | 0.0770 | 0.0377 |
| | carrageenan | 0.3874 | 0.3935 | 0.1927 |
| | pullulan | 8.4211 | 8.5543 | 4.1900 |
| | PVP | 2.5263 | 2.5663 | 1.2570 |
| flavor / color mix | glycerin | 0.4211 | 0.4277 | 0.2095 |
| | polysorbate 80 | 0.3217 | 0.3268 | 0.1601 |
| | atmos 300 | 0.3217 | 0.3268 | 0.1601 |
| | neobee 1053 | 0.6451 | 0.6553 | 0.3210 |
| | cherry flavor | 2.2737 | 2.3097 | 1.1313 |
| | FD&C red #40 | 0.0421 | 0.0428 | 0.0209 |
| Total | | 98.441937 | 100 | 100 |

A fast-dissolving, thin film containing 80 mg of acetaminophen is prepared using a method similar to that described above with respect to Example 1.

### Example 4 - 52% active, 36% polymer

| | **Component** | **mg/film** | **% w/w dry** | **% w/w wet** |
|---|---|---|---|---|
| | purified water | 0 | 0 | 68.3890 |
| | acesulfame K | 0.3030 | 0.3163 | 0.1000 |
| | sucralose | 4.0909 | 4.2707 | 1.3500 |
| | atenolol | 50.0000 | 52.1970 | 16.5000 |
| polymer mix | xanthan gum | 0.1515 | 0.1582 | 0.0500 |
| | locust bean gum | 0.1515 | 0.1582 | 0.0500 |
| | carrageenan | 0.7788 | 0.8130 | 0.2570 |
| | pullulan | 33.1212 | 34.5766 | 10.9300 |
| flavor / color mix | glycerin | 0.7576 | 0.7909 | 0.2500 |
| | polysorbate 80 | 0.6364 | 0.6643 | 0.2100 |
| | atmos 300 | 0.6364 | 0.6643 | 0.2100 |
| | neobee 1053 | 1.3030 | 1.3603 | 0.4300 |
| | citrus flavor | 3.7879 | 3.9543 | 1.2500 |
| | FD&C yellow #6 | 0.0727 | 0.0759 | 0.0240 |
| Total | | 95.790909 | 100 | 100 |

A fast-dissolving, thin film containing 50 mg of atenolol, a beta blocker, is prepared using a method similar to that described above with respect to Example 1.

### Example 5 - 52% active, 36% polymer

| | **Component** | **mg/film** | **% w/w dry** | **% w/w wet** |
|---|---|---|---|---|
| | purified water | 0 | 0 | 68.3890 |
| | acesulfame K | 0.3030 | 0.3163 | 0.1000 |
| | sucralose | 3.6818 | 4.2707 | 1.3500 |
| | ferrous fumarate | 45.000 | 52.1970 | 16.5000 |
| polymer mix | xanthan gum | 0.1364 | 0.1582 | 0.0500 |
| | locust bean gum | 0.1364 | 0.1582 | 0.0500 |
| | carrageenan | 0.7009 | 0.8130 | 0.2570 |
| | pullulan | 29.8091 | 34.5766 | 10.9300 |
| flavor / color mix | glycerin | 0.6818 | 0.7909 | 0.2500 |
| | polysorbate 80 | 0.5727 | 0.6643 | 0.2100 |
| | atmos 300 | 0.5727 | 0.6643 | 0.2100 |
| | neobee 1053 | 1.1727 | 1.3603 | 0.4300 |
| | mint flavor | 3.4091 | 3.9543 | 1.2500 |
| | FD&C yellow #6 | 0.0655 | 0.0759 | 0.0240 |
| Total | | 86.211818 | 100 | 100 |

A fast-dissolving, thin film containing 45 mg of ferrous fumarate, a mineral supplement, is prepared using a method similar to that described above with respect to Example 1.

### Example 6 - 81 % active, 14% polymer

| | **Component** | **mg/film** | **% w/w dry** | **% w/w wet** |
|---|---|---|---|---|
| | purified water | 0 | 0 | 46.0530 |
| | acesulfame K | 0.1538 | 0.1248 | 0.0673 |
| | sucralose | 2.0769 | 1.6852 | 0.9091 |
| | guaifenesin | 100.0000 | 81.1371 | 43.7710 |
| polymer mix | xanthan gum | 0.0770 | 0.0625 | 0.0337 |
| | locust bean gum | 0.0770 | 0.0625 | 0.0337 |
| | carrageenan | 0.3955 | 0.3209 | 0.1731 |
| | HPMC | 0.8225 | 0.6673 | 0.3600 |
| | Pullulan | 15.9930 | 12.9763 | 7.0003 |
| flavor / color mix | glycerin | 0.3847 | 0.3122 | 0.1684 |
| | polysorbate 80 | 0.3230 | 0.2621 | 0.1414 |
| | atmos 300 | 0.3230 | 0.2621 | 0.1414 |
| | neobee 1053 | 0.6616 | 0.5368 | 0.2896 |
| | mint flavor | 1.9232 | 1.5604 | 0.8418 |
| | green #3 | 0.0369 | 0.0300 | 0.0162 |
| Total | | 123.24818 | 100 | 100 |

A fast-dissolving, thin film containing 100 mg of guaifenesin, a mucousloosening agent, is prepared using a method similar to that described above with respect to Example 1.

### Example 7 - 77% active, 16% polymer

| | **Component** | **mg/film** | **% w/w dry** | **% w/w wet** |
|---|---|---|---|---|
| | purified water | 0 | 0 | 49.7678 |
| | acesulfame K | 0.1516 | 0.1465 | 0.0736 |
| | sucralose | 2.7788 | 2.6850 | 1.3487 |
| | acetaminophen | 80.0000 | 77.2987 | 38.8288 |
| polymer mix | xanthan gum | 0.0758 | 0.0732 | 0.0368 |
| | locust bean gum | 0.0758 | 0.0732 | 0.0368 |
| | carrageenan | 0.3874 | 0.3743 | 0.1880 |
| | pullulan | 16.0000 | 15.4597 | 7.7658 |
| flavor / color mix | glycerin | 0.4211 | 0.4068 | 0.2044 |
| | polysorbate 80 | 0.3217 | 0.3108 | 0.1561 |
| | atmos 300 | 0.3217 | 0.3108 | 0.1561 |
| | neobee 1053 | 0.6451 | 0.6233 | 0.3131 |
| | mint flavor | 2.2737 | 2.1969 | 1.1036 |
| | green #3 | 0.0421 | 0.0407 | 0.0204 |
| Total | | 103.49457 | 100 | 100 |

A fast-dissolving, thin film containing about 80 mg of acetaminophen, an analgesic agent, is prepared using a method similar to that described above with respect to Example 1. In this example, the acetaminophen is pre-emulsified with the glycerin and polysorbate 80 in a small portion of water prior to addition to the final mix. The purpose of the pre-mix is to form an emulsifier-active association for better dispersion and taste properties. The term "emulsifier-active association" as used herein means the active is partially or completely coated or surrounded by the emulsifier molecules.

### Example 8 - 50.8% active, 11.7% polymer:

| **Components** | | **mg/film** | **% w/w dry** | **% w/w wet** |
|---|---|---|---|---|
| | | | | |
| Purified Water | | 0.0000 | 0.0000 | 51.0190 |
| Acesulfame K | | 0.1515 | 0.1539 | 0.0754 |
| Sucralose | | 2.7787 | 2.8227 | 1.3826 |
| Taste Masked Ibuprofen 62.5% load | | 80.0000 | 81.2662 | 39.8050 |
| | | | | |

| **Gum Mix** | | | | |
|---|---|---|---|---|
| Xanthan Gum | | 0.0758 | 0.0770 | 0.0377 |
| Locust Bean Gum | | 0.0758 | 0.0770 | 0.0377 |
| Carrageenan | | 0.3873 | 0.3934 | 0.1927 |
| Pullulan | | 8.4211 | 8.5543 | 4.1900 |
| Polyvinylpyrrolidone | | 2.5263 | 2.5663 | 1.2570 |
| | | | | |

| **Flavor Mix** | | | | |
|---|---|---|---|---|
| Glycerin | | 0.4211 | 0.4277 | 0.2095 |
| Polysorbate 80 | | 0.3218 | 0.3269 | 0.1601 |
| Atmos 300 | | 0.3218 | 0.3269 | 0.1601 |
| Neobee 1053 | | 0.6451 | 0.6554 | 0.3210 |
| Mint Flavor | | 2.2737 | 2.3097 | 1.1313 |
| Green #3 | | 0.0420 | 0.0427 | 0.0209 |
| | | | | |
| | Total | 98.441904 | 100.0000 | 100.0000 |

A fast-dissolving, thin film containing 80 mg of taste masked ibuprofen, an analgesic agent, is prepared using a method similar to that described above with respect to Example 1. The particles used in this example have been coated with fats and waxes by the spinning disk approach prior to making the film to provide taste-masking. The coating process (also referred to in Example 2) can be any conventional type granulation or encapsulation processes. Examples include, but not limited to fluidized bed granulation, hot-melt extrusion, spinning disk encapsulation, phase-separation type encapsulation.

### Example 9 - 49.8%, 36% polymer

| **Components** | | **mg/film** | **% w/w dry** | **% w/w wet** |
|---|---|---|---|---|
| Purified Water | | 0.0000 | 0.0000 | 59.1445 |
| Acesulfame K | | 0.1351 | 0.1224 | 0.0500 |
| Sucralose | | 4.8649 | 4.4058 | 1.8000 |
| Ibuprofen | | 50.0000 | 45.2815 | 18.5000 |
| Sustained release Phenylephrine HCl particles 50% load | | 10.0000 | 9.0563 | 3.7000 |
| | | | | |

| **Gum Mix** | | | | |
|---|---|---|---|---|
| Xanthan Gum | | 0.0911 | 0.0825 | 0.0337 |
| Locust Bean Gum | | 0.0911 | 0.0825 | 0.0337 |
| Carrageenan | | 0.4678 | 0.4237 | 0.1731 |
| Hydroxylpropylmethyl cellulose | | 5.4054 | 4.8953 | 2.0000 |
| Pullulan | | 33.7838 | 30.5956 | 12.5000 |
| | | | | |

| **Flavor Mix** | | | | |
|---|---|---|---|---|
| Glycerin | | 0.6757 | 0.6119 | 0.2500 |
| Polysorbate 80 | | 0.3649 | 0.3304 | 0.1350 |
| Atmos 300 | | 0.3649 | 0.3304 | 0.1350 |
| Neobee 1053 | | 0.7297 | 0.6609 | 0.2700 |
| Mint Flavor | | 3.3784 | 3.0596 | 1.2500 |
| Green #3 | | 0.0676 | 0.0612 | 0.0250 |
| | Total | 110.42027 | 100 | 100.0000 |

A fast-dissolving, thin film containing 50 mg of ibuprofen and 10 mg of sustained release phenylephrine HCl (5mg phenylephrine HCl) is prepared using a method similar to that described above with respect to Example 1. The phenylephrine is coated with a combination of ethylcellulose and hydroxypropyl methylcellulose using the fluidized bed approach as described in Example 8 to provide sustained release properties. As illustrated, the film may contain a combination of active ingredients, for instance, as illustrated in this example, ibuprofen and sustained release phenylephrine particles, rendering the resulting film containing a sustained release (or controlled released) properties.

### Example 10 - 49.8% active, 36% polymer.

| **Components** | | **mg/film** | **% w/w dry** | **% w/w wet** |
|---|---|---|---|---|
| Purified Water | | 0.0000 | 0.0000 | 59.1445 |
| Acesulfame K | | 0.1351 | 0.1224 | 0.0500 |
| Sucralose | | 4.8649 | 4.4058 | 1.8000 |
| Ibuprofen | | 50.0000 | 45.2815 | 18.5000 |
| Sustained release Phenylephrine HCl particles 50% load | | 10.0000 | 9.0563 | 3.7000 |
| | | | | |

| **Gum Mix** | | | | |
|---|---|---|---|---|
| Xanthan Gum | | 0.0911 | 0.0825 | 0.0337 |
| Locust Bean Gum | | 0.0911 | 0.0825 | 0.0337 |
| Carrageenan | | 0.4678 | 0.4237 | 0.1731 |
| Hydroxylpropylmethyl cellulose | | 5.4054 | 4.8953 | 2.0000 |
| Pullulan | | 33.7838 | 30.5956 | 12.5000 |
| | | | | |

| **Flavor Mix** | | | | |
|---|---|---|---|---|
| Glycerin | | 0.6757 | 0.6119 | 0.2500 |
| Polysorbate 80 | | 0.3649 | 0.3304 | 0.1350 |
| Atmos 300 | | 0.3649 | 0.3304 | 0.1350 |
| Neobee 1053 | | 0.7297 | 0.6609 | 0.2700 |
| Mint Flavor | | 3.3784 | 3.0596 | 1.2500 |
| Green #3 | | 0.0676 | 0.0612 | 0.0250 |
| | Total | 110.42027 | 100 | 100.0000 |

A fast-dissolving, thin film containing 50 mg of ibuprofen and 10 mg sustained release phenylephrine HCl (5mg phenylephrine HCl) is prepared using a method similar to that described above with respect to Example 1. The phenylephrine is coated with a combination of ethylcellulose and hydroxypropyl methylcellulose using the fluid be granulator using fluidized bed approach referred to in Example 8 to provide controlled release properties. As illustrated in this example and example 9, although the film itself is a quick-dissolve film, the pharmacological action of the active delivered may be sustained or delayed and/or additionally targeted to a specific section of the gastrointestinal tract. The particles could be coated with polymethacrylates using a typical fluidized bed granulator and rendering the coating to disintegrate at the latter portion of the gastrointestinal tract when the pH is higher than 5.

## Claims

1. A fast-dissolving or fast-disintegrating film having a thickness of between about 25 microns and about 250 microns and comprising:
an active agent comprising at least about 70% of the dry film, by weight; and
at least one water-soluble polymer comprising between about 10% and about 40% of the film, by weight;
wherein pullulan is the sole or primary water-soluble polymer; and
wherein the active agent is a poorly-water-soluble solid at room temperature.

2. The film of claim 1, wherein the active agent includes at least one active selected from the group consisting of a pharmaceutical, a cosmetic agent, a vitamin, a mineral or a mixture thereof.

3. The film of claim 1, wherein the active agent comprises between 70% and 90% of the film, by weight, or
wherein the active agent comprises between 70% and 80% of the film, by weight.

4. The film of any preceding claim, wherein the at least one water-soluble polymer comprises between about 10% and about 30% of the film, by weight, or
wherein the at least one water-soluble polymer comprises about 12% to about 18% of the film, by weight.

5. The film of any preceding claim, wherein the film is orally-consumable.

6. The film of claim 5, wherein the film is adapted to substantially dissolve or disassociate within an oral cavity within about 1 minute.

7. The film of claim 5, further comprising an agent selected from the group consisting of a flavoring agent; a coloring agent and a mixture thereof.

8. The film of any one of claims 1 to 4, wherein the film is for topical administration.

9. The film of any preceding claim, wherein the film is a multi-layer film.

10. The film of any preceding claim, wherein the film is substantially free of modified starches.

11. The film of claim 2, wherein the pullulan comprises about 15% of the film, by weight.

12. A method for preparing a fast-dissolving or fast-disintegrating film having a thickness of between about 25 microns and about 250 microns and comprising a high dose of active agent, the method comprising the steps of:
dissolving in a quantity of water or a quantity of water and an alcohol, a quantity of at least one water-soluble polymer comprising between about 10% and about 40% of the dry film, by weight, wherein pullulan is the sole or primary water-soluble polymer;
adding to the resulting solution a quantity of at least one active agent comprising at least about 70% of the dry film, by weight, wherein the at least one active agent is a poorly-water-soluble solid at room temperature;
casting the resulting suspension on a supporting substrate; and
drying the cast suspension to form a film.

13. The method of claim 12, wherein the at least one water-soluble polymer comprises between about 10% and about 30% of the film, by weight, or
wherein the at least one water-soluble polymer comprises about 15% of the film, by weight.

14. The method of claim 12 or claim 13, wherein the at least one active agent is selected from a group consisting of: a pharmaceutical, a medicament, a drug, a therapeutic agent, a diagnostic agent, a cosmetic agent, a nutritional supplement and a mixture thereof.

15. The method of any one of claims 12 to 14, wherein the active agent comprises between 70% and 90% of the film, by weight, or
wherein the active agent comprises between 70% and 80% of the film, by weight.

16. The method of any one of claims 12 to 15, further comprising the step of:
cutting the dried film to a size sufficient to contain a desired dose of the active agent.

17. The method of any one of claims 12 to 16, wherein the film is for oral or topical administration.

18. The film of claim 1, wherein the active agent is acetaminophen.

## Patentansprüche

1. Schnellauflösender oder schnellzerfallender Film mit einer Dicke zwischen etwa 25 Mikrometer und etwa 250 Mikrometer, umfassend:
einen Wirkstoff, der wenigstens etwa 70 Gew.-% des trockenen Films bildet; und
wenigstens ein wasserlösliches Polymer, das zwischen etwa 10 Gew.-% und etwa 40 Gew.-% des Films bildet;
wobei Pullulan das einzige oder hauptsächliche wasserlösliche Polymer ist; und
wobei der Wirkstoff ein bei Raumtemperatur schlecht wasserlöslicher Feststoff ist.

2. Film gemäß Anspruch 1, wobei der Wirkstoff wenigstens einen Wirkstoff ausgewählt aus der Gruppe bestehend aus einem Pharmazeutikum, einem kosmetischen Mittel, einem Vitamin, einem Mineralstoff oder einem Gemisch davon umfasst.

3. Film gemäß Anspruch 1, wobei der Wirkstoff zwischen 70 Gew.-% und 90 Gew.-% des Films bildet oder
wobei der Wirkstoff zwischen 70 Gew.-% und 80 Gew.-% des Films bildet.

4. Film gemäß einem der vorstehenden Ansprüche, wobei das wenigstens eine wasserlösliche Polymer zwischen etwa 10 Gew.-% und etwa 30 Gew.-% des Films bildet oder
wobei das wenigstens eine wasserlösliche Polymer etwa 12 Gew.-% bis etwa 18 Gew.-% des Films bildet.

5. Film gemäß einem der vorstehenden Ansprüche, wobei der Film oral aufnehmbar ist.

6. Film gemäß Anspruch 5, wobei der Film dafür ausgelegt ist, sich in einer Mundhöhle innerhalb von etwa 1 Minute wesentlich zu lösen oder aufzulösen.

7. Film gemäß Anspruch 5, ferner umfassend ein Mittel ausgewählt aus der Gruppe bestehend aus einem Aromastoff, einem Farbstoff und einem Gemisch davon.

8. Film gemäß einem der Ansprüche 1 bis 4, wobei der Film für die topische Verabreichung ist.

9. Film gemäß einem der vorstehenden Ansprüche, wobei der Film ein mehrschichtiger Film ist.

10. Film gemäß einem der vorstehenden Ansprüche, wobei der Film im Wesentlichen frei von modifizierten Stärken ist.

11. Film gemäß Anspruch 2, wobei das Pullulan etwa 15 Gew.-% des Films bildet.

12. Verfahren zum Herstellen eines schnellauflösenden oder schnellzerfallenden Films mit einer Dicke zwischen etwa 25 Mikrometer und etwa 250 Mikrometer und umfassend eine hohe Dosis an Wirkstoff, wobei das Verfahren folgende Schritte umfasst:
Auflösen einer Menge von wenigstens einem wasserlöslichen Polymer, die zwischen etwa 10 Gew.-% und etwa 40 Gew.-% des trockenen Films bildet, worin Pullulan das einzige oder hauptsächliche wasserlösliche Polymer ist, in einer Menge an Wasser oder einer Menge an Wasser und an einem Alkohol;
Zugeben einer Menge an wenigstens einem Wirkstoff, die wenigstens etwa 70 Gew.-% des trockenen Films bildet, zu der erhaltenen Lösung, wobei der wenigstens eine Wirkstoff ein bei Raumtemperatur schlecht wasserlöslicher Feststoff ist;
Gießen der erhaltenen Suspension auf ein tragendes Substrat; und
Trocknen der gegossenen Suspension, um einen Film zu bilden.

13. Verfahren gemäß Anspruch 12, wobei das wenigstens eine wasserlösliche Polymer zwischen etwa 10 Gew.-% und etwa 30 Gew.-% des Films bildet, oder
wobei das wenigstens eine wasserlösliche Polymer etwa 15 Gew.-% des Films bildet.

14. Verfahren gemäß Anspruch 12 oder Anspruch 13, wobei der wenigstens eine Wirkstoff ausgewählt ist aus der Gruppe bestehend aus: einem Pharmazeutikum, einem Medikament, einem Arzneimittel, einem therapeutischen Mittel, einem diagnostischen Mittel, einem kosmetischen Mittel, einer Nahrungsergänzung und einem Gemisch davon.

15. Verfahren gemäß einem der Ansprüche 12 bis 14, wobei der Wirkstoff zwischen 70 Gew.-% und 90 Gew.-% des Films bildet oder
wobei der Wirkstoff zwischen 70 Gew.-% und 80 Gew.-% des Films bildet.

16. Verfahren gemäß einem der Ansprüche 12 bis 15, ferner umfassend den Schritt:
Schneiden des getrockneten Films auf eine Größe, die ausreicht, um eine gewünschte Dosis des Wirkstoffs zu enthalten.

17. Verfahren gemäß einem der Ansprüche 12 bis 16, wobei der Film für die orale oder topische Verabreichung ist.

18. Film gemäß Anspruch 1, wobei der Wirkstoff Acetaminophen ist.

## Revendications

1. Film à solubilisation rapide ou à désintégration rapide ayant une épaisseur comprise entre environ 25 microns et environ 250 microns, et comprenant :
un agent actif constituant au moins environ 70% du film sec, en poids ; et
au moins un polymère hydrosoluble constituant entre environ 10% et environ 40% du film, en poids ;
où le pullulane est le polymère hydrosoluble seul ou principal ; et
où l'agent actif est un solide peu soluble dans l'eau à température ambiante.

2. Film selon la revendication 1, **caractérisé en ce que** l'agent actif comporte au moins une substance active choisie dans le groupe constitué par une substance pharmaceutique, un agent cosmétique, une vitamine, un minéral ou un mélange de ceux-ci.

3. Film selon la revendication 1, **caractérisé en ce que** l'agent actif constitue entre 70% et 90% du film, en poids, ou
**caractérisé en ce que** l'agent actif constitue entre 70% et 80% du film, en poids.

4. Film selon l'une quelconque des revendications précédentes, **caractérisé en ce que** le au moins un polymère hydrosoluble constitue entre environ 10% et environ 30% du film, en poids, ou
**caractérisé en ce que** le au moins un polymère hydrosoluble constitue d'environ 12% à environ 18% du film, en poids.

5. Film selon l'une quelconque des revendications précédentes, **caractérisé en ce que** le film est consommable par voie orale.

6. Film selon la revendication 5, **caractérisé en ce que** le film est adapté pour sensiblement se solubiliser ou se dissocier au sein d'une cavité orale en environ 1 minute.

7. Film selon la revendication 5, comprenant en outre un agent choisi dans le groupe constitué par un agent aromatisant, un agent colorant, et un mélange de ceux-ci.

8. Film selon l'une quelconque des revendications 1 à 4, **caractérisé en ce que** le film est destiné à une administration topique.

9. Film selon l'une quelconque des revendications précédentes, **caractérisé en ce que** le film est un film multicouches.

10. Film selon l'une quelconque des revendications précédentes, **caractérisé en ce que** le film est sensiblement dépourvu d'amidons modifiés.

11. Film selon la revendication 2, **caractérisé en ce que** le pullulane constitue environ 15% du film, en poids.

12. Méthode de préparation d'un film à solubilisation rapide ou à désintégration rapide ayant une épaisseur comprise entre environ 25 microns et environ 250 microns, et comprenant une dose élevée d'agent actif, la méthode comprenant les étapes consistant à :
solubiliser dans une quantité d'eau ou une quantité d'eau et d'un alcool, une quantité d'au moins un polymère hydrosoluble constituant entre environ 10% et environ 40% du film sec, en poids, où le pullulane est le polymère hydrosoluble seul ou principal ;
ajouter à la solution résultante une quantité d'au moins un agent actif constituant au moins environ 70% du film sec, en poids, où le au moins un agent actif est un solide peu soluble dans l'eau à température ambiante ;
couler la suspension résultante sur un substrat de support ; et
sécher la suspension coulée afin de former un film.

13. Méthode selon la revendication 12, **caractérisée en ce que** le au moins un polymère hydrosoluble constitue entre environ 10% et environ 30% du film, en poids, ou
**caractérisée en ce que** le au moins un polymère hydrosoluble constitue environ 15% du film, en poids.

14. Méthode selon la revendication 12 ou la revendication 13, **caractérisée en ce que** le au moins un agent actif est choisi dans le groupe constitué par une substance pharmaceutique, un médicament, une drogue, un agent thérapeutique, un agent de diagnostic, un agent cosmétique, un complément nutritionnel, et un mélange de ceux-ci.

15. Méthode selon l'une quelconque des revendications 12 à 14, **caractérisée en ce que** l'agent actif constitue entre 70% et 90% du film, en poids, ou
**caractérisée en ce que** l'agent actif constitue entre 70% et 80% du film, en poids.

16. Méthode selon l'une quelconque des revendications 12 à 15, comprenant en outre l'étape consistant à
découper le film séché jusqu'à une taille suffisante afin de contenir une dose désirée de l'agent actif.

17. Méthode selon l'une quelconque des revendications 12 à 16, **caractérisée en ce que** le film est destiné à une administration orale ou topique.

18. Film selon la revendication 1, **caractérisé en ce que** l'agent actif est l'acétaminophène.
